# EUROPÄISCHE PATENTSCHRIFT

(11) **EP 0 826 338 B1**
(45) Veröffentlichungstag und Bekanntmachung des Hinweises auf die Patenterteilung: **28.05.2003**
(21) Anmeldenummer: 97113653.6
(22) Anmeldetag: 07.08.1997
(51) Int. Cl.: A61B 17/06

(54) **Mehrfäden-Packung für chirurgisches Nahtmaterial**
Packaging for multiple sutures
Emballage pour multiples sutures

(30) Priorität: 28.08.1996 DE 19634726
(43) Veröffentlichungstag der Anmeldung: 04.03.1998
(73) Patentinhaber: Aesculap AG & Co. KG, 78532 Tuttlingen (DE)
(72) Erfinder: Odermatt, Erich K., Dr., 08191 Rubi (ES)
(74) Vertreter: Selting, Günther, Dipl.-Ing.

(56) Entgegenhaltungen:
- EP-A- 0 065 098
- EP-A- 0 505 612
- US-A- 4 572 363

## Beschreibung

Die Erfindung betrifft eine Mehrfäden-Packung für chirurgisches Nahtmaterial, in der mehrere Fäden enthalten sind, die jeweils mit mindestens einer Nadel verbunden sind.

Aus EP 0 065 098 B1 ist eine Mehrfäden-Packung für chirurgisches Nahtmaterial bekannt, die eine Faltkarte aufweist, welche die Hülle der Packung bildet. Eine erste Platte der Faltkarte dient als Wickelplatte. Diese hat mehrere Löcher, durch die Dorne hindurchgesteckt werden können, um die herum ein Faden gewickelt wird. Ferner ist an der Wickelplatte eine Nadelhaltevorrichtung in Form eines Schaumstoffstreifens mit mehreren Schlitzen angebracht. In die Schlitze des Schaumstoffstreifens können einzeln die an den Fäden befestigten Nadeln eingesteckt werden. Nach dem Wickeln des ersten Fadens auf der ersten Platte wird auf die erste Platte eine Trennplatte aufgelegt, so daß der gewickelte Faden überdeckt wird. Diese Trennplatte hat ebenfalls Löcher, die mit denjenigen der ersten Platte fluchten, so daß die Wickeldorne hindurchgesteckt werden können. Auf der Trennplatte wird dann ein weiterer Faden durch Wickeln um die Wickeldorne herum verlegt. Danach können weitere Trennplatten aufgelegt werden. Der Stapel von Trennplatten mit den dazwischenliegenden Fadenwickeln wird dann durch übergefaltete weitere Platten bedeckt, die zusammen mit der ersten Platte ein Behältnis bilden. Die Nadeln sämtlicher Fäden werden an der Nadelhaltevorrichtung fixiert. Zusätzlich kann in die Tasche ein separater Nahtmaterialträger mit eigener Nadelhaltevorrichtung eingesetzt werden, der nach dem Öffnen der Packung weitere Nadeln präsentiert. Anstelle des Stapels separater Trennplatten kann auch eine ziehharmonikaartige Struktur aus zusammenhängenden Trennplatten benutzt werden. In jedem Fall sind die Trennplatten Zusatzteile, die an dem Kartenzuschnitt befestigt werden müssen und bewirken, daß die Packung sehr dick wird.

EP 0 505 612 B1 beschreibt eine Mehrfäden-Packung aus zahlreichen nebeneinander angeordneten Platten, die ein komplexes Faltpaket bilden und seitliche Aussparungen für Wickeldorne aufweisen. Auf jede Platte kann ein Faden gewickelt werden, der dann durch Überfalten einer benachbarten Platte bedeckt wird. Anschließend wird auf der benachbarten Platte ein weiterer Faden gewickelt. Die Nadeln sämtlicher Fäden sind in einer Nadelhaltevorrichtung zusammengefaßt, die mit der ersten Wickelplatte faltbar verbunden ist. Die Nadeln werden durch ein Fenster der zweiten Platte hindurchgeführt, damit sie später von außen ergriffen werden können. Die Nadeln liegen also nicht insgesamt zum Ergreifen frei, sondern nur mit den aus dem Fenster herausragenden Nadelteilen.

US-A-4,572,363, von der der Oberbegriff des Patentanspruchs 1 ausgeht, beschreibt eine Mehrfäden-Packung aus einem einstückigen Zuschnitt, der mehrere übereinander faltbare Platten aufweist, von denen eine eine Nadelhaltevorrichtung trägt. Zusätzlich sind zwei separate Einsatzteile vorgesehen. Das eine Einsatzteil besteht lediglich aus einer ersten Platte. Das andere Einsatzteil besteht aus drei faltbar miteinander verbundenen Trennplatten, die eine L-förmige Struktur bilden. Auch diese Packung ist wegen der Notwendigkeit, zahlreiche Trennplatten übereinander zu legen, sehr dick. Außerdem können die Einsatzteile sich von dem Zuschnitt lösen.

Der Erfindung liegt die Aufgabe zugrunde, eine Mehrfäden-Packung für chirurgisches Nahtmaterial zu schaffen, die mit wenig Packungsmaterial die Unterbringung einer großen Zahl von Fäden ermöglicht.

Die Lösung dieser Aufgabe erfolgt erfindungsgemäß mit den im Patentanspruch 1 angegebenen Merkmalen.

Die erfindungsgemäße Mehrfäden-Packung weist eine als Wickelplatte oder Grundplatte dienende erste Platte auf, die an gegenüberliegenden Kanten mit Trennplatten verbunden ist, welche über die erste Platte umgefaltet werden können. Auf diese Weise können über der ersten Platte nacheinander mehrere Fäden gewickelt und abgelegt werden, die anschließend durch Umfalten einer der Trennplatten überdeckt werden, wobei das Wickeln des nächsten Fadens über dieser Trennplatte erfolgt. Die Trennplatten können abwechselnd von rechts und von links über die erste Platte gefaltet werden. Diese Packung kann in eine damit verbundene oder separate Tasche eingesetzt werden.

Gemäß einer bevorzugten Ausführungsform der Erfindung ist vorgesehen, dass an jeder der beiden entgegengesetzten Kanten der ersten Platte mindestens zwei Trennplatten angeordnet sind, wobei jeweils mindestens zwei einander gegenüberliegende Trennplatten auf der ersten Platte übereinandergefaltet sind. Hierbei wird von der Überlegung ausgegangen, daß es nicht erforderlich ist, daß jede Trennplatte die erste Platte vollständig bedeckt. Vielmehr reicht es aus, wenn eine Trennplatte nur einen Teil der ersten Platte bedeckt und eine andere Trennplatte einen weiteren Teil der ersten Platte bedeckt. Damit kann das durch eine Trennplatte bedeckte Nahtmaterial in definierter Weise unter dieser Trennplatte festgehalten werden, während über der Trennplatte ein anderer Faden gewickelt wird. Es reicht aus, einen Teil des Fadenwickels mit der Trennplatte abzudecken, ohne daß die Gefahr besteht, daß der freiliegende Teil des Fadenwickels sich mit einem benachbarten Fadenwickel verwirrt. Dadurch wird eine erhebliche Materialersparnis an Plattenmaterial erreicht.

Bei einer anderen Ausführungsform der Erfindung erstrecken sich die Trennplatten jeweils über Teile der Breite der ersten Platte, z.B. die halbe Breite, und jede Trennplatte ist mit einer Rückfaltplatte verbunden. Dabei kann unter jeder Trennplatte und über derselben Trennplatte (jedoch unter der Rückfaltplatte) jeweils ein Fadenwickel abgelegt werden. Die Trennplatten bilden einerseits mit der ersten Platte und andererseits mit der Rückfaltplatte eine Tasche, die einen Fadenwickel aufnimmt.

Es besteht die Möglichkeit, die Mehrfäden-Packung so auszubilden, daß die erste Platte mit einer integrierten Tasche einstückig verbunden ist. Hierzu ist die erste Platte mit einer zweiten Platte verbunden, die über die auf die erste Platte gefalteten Trennplatten gefaltet werden kann.

Alternativ besteht die Möglichkeit, eine zusätzliche Tasche vorzusehen, die aus einem separaten Zuschnitt besteht und in die die Packung als Nahtmaterialträger eingebracht wird. Nach dem Öffnen der Tasche liegt die Nadelhaltevorrichtung der Packung frei. Die Packung kann in die Tasche lose eingelegt oder auch fixiert werden. Gemäß einer bevorzugten Ausführungsform der Erfindung sind sowohl die Tasche als auch die Packung trapezförmig gestaltet, wobei ihre Breite zur Taschenöffnung hin abnimmt. Dies hat zur Folge, daß die Pakkung in der Tasche fixiert ist und durch die Taschenöffnung nicht herausgezogen werden kann. Zum Entnehmen der Fäden ist es lediglich erforderlich, eine Deckplatte zu öffnen, um die Taschenöffnung und die dann freiliegenden Nadelhaltevorrichtungen zugänglich zu machen.

Im folgenden werden unter Bezugnahme auf die Zeichnungen Ausführungsbeispiele der Erfindung näher erläutert.

Es zeigen:
- Fig. 1: den Zuschnitt einer Mehrfäden-Packung im auseinandergeklappten Zustand nach dem Herstellen zweier Fadenwickel,
- Fig. 2: die Packung nach Fig. 1 nach dem Umklappen einer Trennplatte und dem Herstellen eines zweiten Fadenwickels über dieser Trennplatte,
- Fig. 3: dieselbe Packung nach dem Umklappen der zweiten Trennplatte und dem Herstellen zweier dritter Fadenwickel,
- Fig. 4: die Packung nach dem Umklappen der dritten Trennplatte und dem Herstellen eines vierten Fadenwickels,
- Fig. 5: die Packung nach dem Umklappen (Schließen) der vierten Trennplatte,
- Fig. 6: eine Stirnansicht der Packung nach Fig. 5 aus Richtung des Pfeiles VI mit einer herausziehbaren Zweitlage,
- Fig. 7: eine Stirnansicht der nahezu geschlossenen Packung mit einer herausziehbaren Zweitlage,
- Fig. 8: eine Rückansicht der Packung nach Fig. 7 aus Richtung des Pfeiles VIII,
- Fig. 9: eine Stirnansicht ähnlich derjenigen von Fig. 7 bei einem anderen Ausführungsbeispiel mit Präsentation von zwei Nadelreihen,
- Fig. 10: den Zuschnitt eines separaten Nahtmaterialträgers,
- Fig. 11: das Schließen des weiteren Nahtmaterialträgers,
- Fig. 12: ein anderes Ausführungsbeispiel der Packung nach dem Herstellen zweier erster Fadenwickel mit 8er- und Mäanderwickel,
- Fig. 13: eine Stirnansicht der Packung nach Fig. 12 mit eingesetzten Wickeldornen,
- Fig. 14: das Umfalten der Trennplatten,
- Fig. 15: das Umfalten der Rückfaltplatten,
- Fig. 16: eine Darstellung der Packung nach Fig. 12 nach dem Herstellen und Bedecken der Fadenwickel,
- Fig. 17: das Schließen der Packung nach Fig. 16,
- Fig. 18: eine Rückansicht der geschlossenen Packung nach Fig. 17,
- Fig. 19: einen Nahtmaterialträger, der in eine separate Tasche eingebracht wird, die im aufgefalteten Zustand dargestellt ist,
- Fig. 20: das Schließen der Tasche bei einem eingesetzten Nahtmaterialträger,
- Fig. 21: die Packung im geschlossenen Zustand und
- Fig. 22: das Entnehmen eines Fadens aus der Tasche, deren Deckplatte geöffnet wurde.

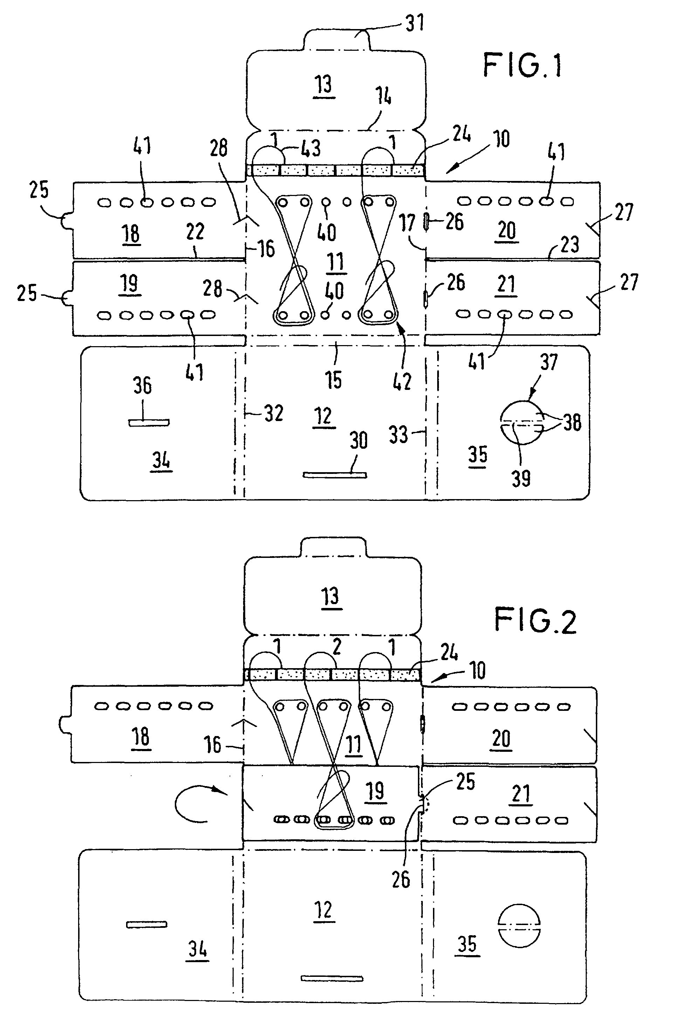

Die Mehrfäden-Packung 10 des Ausführungsbeispiels der Fign. 1-8 besteht aus einem einstückigen Kartonzuschnitt mit einer rechteckigen ersten Platte 11, an deren eine Längskante sich eine rechteckige zweite Platte 12 anschließt. Der zweiten Platte 12 gegenüber befindet sich eine Deckplatte 13, die um eine Faltlinie 14 herumgefaltet werden kann. Die zweite Platte 12 ist mit der ersten Platte 11 durch einen von zwei Faltlinien begrenzten Querstreifen 15 verbunden. Die erste Platte 11, die zweite Platte 12 und die Deckplatte 13 sind jeweils an den Faltlinien von gleicher Breite und sie bilden einen längslaufenden Materialstreifen.

Die erste Platte 11 ist durch längslaufende Faltlinien 16,17 seitlich begrenzt. An die Faltlinie 16 schließen sich zwei Trennplatten 18,19 an und an die Faltlinie 17 schließen sich ebenfalls zwei Trennplatten 20,21 an. Jede dieser Trennplatten hat eine Breite, die im wesentlichen gleich derjenigen der ersten Platte 11 ist. Die Trennplatten 18,19 sind durch einen querlaufenden Schlitz 22 voneinander getrennt und die Trennplatten 20,21 sind ebenfalls durch einen querlaufenden Schlitz 23 voneinander getrennt. Die Trennplatten 18 und 20 können über dem oberen Bereich der ersten Platte 11 übereinandergefaltet werden und die Trennplatten 19 und 21 können über den unteren Bereich der ersten Platte 11 übereinandergefaltet werden.

Oberhalb desjenigen Bereichs der ersten Platte 11, der von den Trennplatten 18-21 bedeckt werden kann, befindet sich eine Nadelhaltevorrichtung 24 in Form eines Schaumstoffstreifens, in dem quer- oder schräglaufende Schlitze vorgesehen sind, in welche die Nadeln von Nahtmaterialfäden eingesetzt werden können, um die Nadeln dort zu fixieren.

An den von der ersten Platte 11 abgewandten Rändern der Trennplatten 18,19 befinden sich Laschen 25, welche in Schlitze 26, die längs der gegenüberliegenden Faltlinie 17 vorgesehen sind, eingesteckt werden können, um die Trennplatten 18,19 im Schließzustand zu fixieren.

Die Platten 20,21 haben an ihren äußeren Längskanten schräge Schlitze 27, die mit umgekehrt-V-förmigen Schlitzen 28 an der Faltlinie 16 zusammengreifen können, um die Trennplatten 20,21 im Schließzustand zu fixieren.

Die zweite Platte 12 ist mit einem Einsteckschlitz 30 versehen, in den eine Lasche 31 der Deckplatte 13 eingesteckt werden kann, um die Packung im Schließzustand zu fixieren.

Die zweite Platte 12 ist durch längslaufende Faltlinien 32,33 begrenzt, an die sich jeweils eine Verschlußplatte 34 bzw. 35 anschließt. Die Verschlußplatte 34 hat einen Querschlitz 36 und die Verschlußplatte 35 ist mit einem Verriegelungselement 37 versehen, das durch den Querschlitz 36 hindurchgesteckt und hinter diesem verriegelt werden kann. Dieses verriegelungselement 37 besteht aus zwei halbkreisförmigen Klappen 38, die zu beiden Seiten eines Steges 39 angeordnet sind und um den Steg 39 herumgeklappt werden können. Im aufgeklappten Zustand liegen die Klappen 38 aneinander und sie können dann durch den Schlitz 36 hindurchgesteckt werden. An der ersten Platte 11 sind zwei, in diesem Falle querlaufende Reihen von Löchern 40 für den Durchtritt von Wickeldornen vorgesehen. Jede der Trennplatten 18-21 hat ebenfalls eine Reihe von Löchern 41. Wenn die Trennplatte um die Faltlinie 16 bzw. 17 herum auf die erste Platte 11 herumgefaltet worden ist, liegen die Löcher 41 der Trennplatte deckungsgleich über den Löchern 40 der ersten Platte 11. Damit das Umfalten durch die Wickeldorne nicht beeinträchtigt wird, sind die Löcher 41 als Langlöcher ausgebildet, die ihre größte Länge in Querrichtung haben.

Die gemäß Fig. 1 ausgebreitete Faltkarte wird auf eine Gruppe von Wickeldornen aufgesetzt, die durch die Löcher 40 hindurchragen. Dann werden maschinell oder von Hand die Fadenwickel 42 aus chirurgischem Fadenmaterial gewickelt. Im vorliegenden Fall ist eine 8-förmige Wicklung vorgesehen, jedoch können auch O- oder mäanderförmige Wicklungen gewickelt werden. Jeder Nahtmaterialwickel hat mindestens eine bogenförmige Nadel 43, die mit dem Faden fest verbunden ist. Diese Nadel 43 wird an der aus Schaumstoff bestehenden Nadelhaltevorrichtung 43 befestigt, so daß sie später mit der Hand oder einem Instrument ergriffen werden kann, um den betreffenden Faden aus der Packung zu ziehen.

In den Fign. 1-4 sind die Fadenwickel 42 jeweils in der Reihenfolge ihres Herstellens mit 1 bis 4 bezeichnet.

Gemäß Fig. 1 werden zwei erste Fadenwickel 1 unmittelbar auf der ersten Platte 11 gewickelt. Damit diese Fadenwickel sich gegenseitig nicht stören, haben sie einen gegenseitigen Abstand. Jede Schleife eines Fadenwickels ist um zwei Löcher bzw. zwei durch die Löcher 40 hindurchgesteckte Wickeldorne, gewickelt, wobei zwischen den beiden Fadenwickeln zwei Löcher 40 unbenutzt bleiben, damit die Fadenwickel den erforderlichen gegenseitigen Abstand haben.

Nach dem Herstellen der ersten Fadenwickel 1 wird eine der Platten 18,19, im vorliegenden Fall die Trennplatte 19, um die Faltlinie 16 herum über den unteren Bereich der ersten Platte 11 gefaltet, wobei die Lasche 25 in den Schlitz 26 eingesteckt wird. Dabei überdeckt die Trennplatte 19 die unteren Hälften der bereits hergestellten ersten Fadenwickel 1. Dann wird zwischen den ersten Fadenwicklen 1 ein zweiter Fadenwickel 2 gewikkelt und zwar in einem Bereich, der zwischen den ersten Fadenwickeln 1 noch frei ist. Die Nadeln 32 aller Fadenwickel werden an derselben Nadelhaltevorrichtung 24 befestigt.

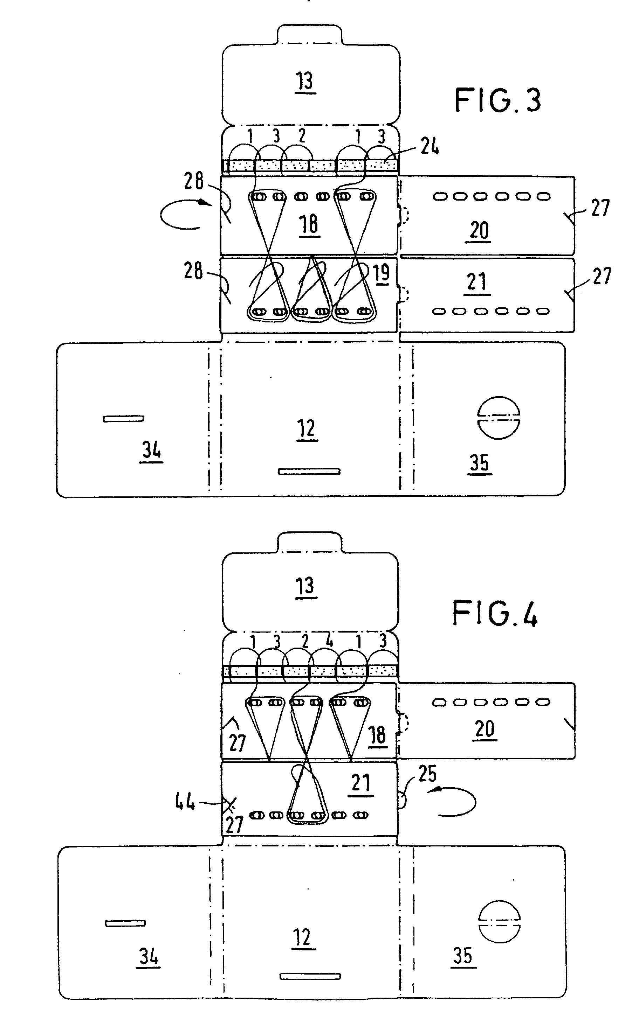

Nach dem Herstellen des zweiten Fadenwickels 2 wird die obere linke Trennplatte 18 umgefaltet, so daß sie die oberen Hälften der (drei) bereits fertigen Fadenwickel überdeckt. Dann werden gemäß Fig. 3 zwei (dritte) Fadenwickel 3 über den beiden Trennplatten 18,19 gewikkelt, und zwar genau über den beiden ersten Fadenwikkeln 2. Auf der unteren Trennplatte 19 behindern die dritten Fadenwickel 3 den zweiten Fadenwickel 2 nicht, weil dieser bereits zur Hälfte unter der oberen Trennplatte 18 fixiert ist. Die dritten Fadenwickel 3 haben einen gegenseitigen Abstand voneinander und behindern sich ebenfalls nicht.

Danach wird gemäß Fig. 4 die rechte untere Trennplatte 21 auf die linke untere Trennplatte 19 gefaltet und dadurch fixiert, daß die von dem V-förmigen Schlitz 28 gebildete Spitze 44 in den Schrägschlitz 27 der Trennplatte 21 eingesteckt wird. Nun wird über den Platten 18,21 ein vierter Fadenwickel 4 verlegt, und zwar zwischen den beiden dritten Fadenwickeln 3. Die letzte Trennplatte 20 bedeckt den oberen Teil des vierten Fadenwickels 4. Sie wird mit ihrem Schrägschlitz 27 hinter der von dem Doppelschlitz 28 gebildeten Spitze 44 verriegelt.

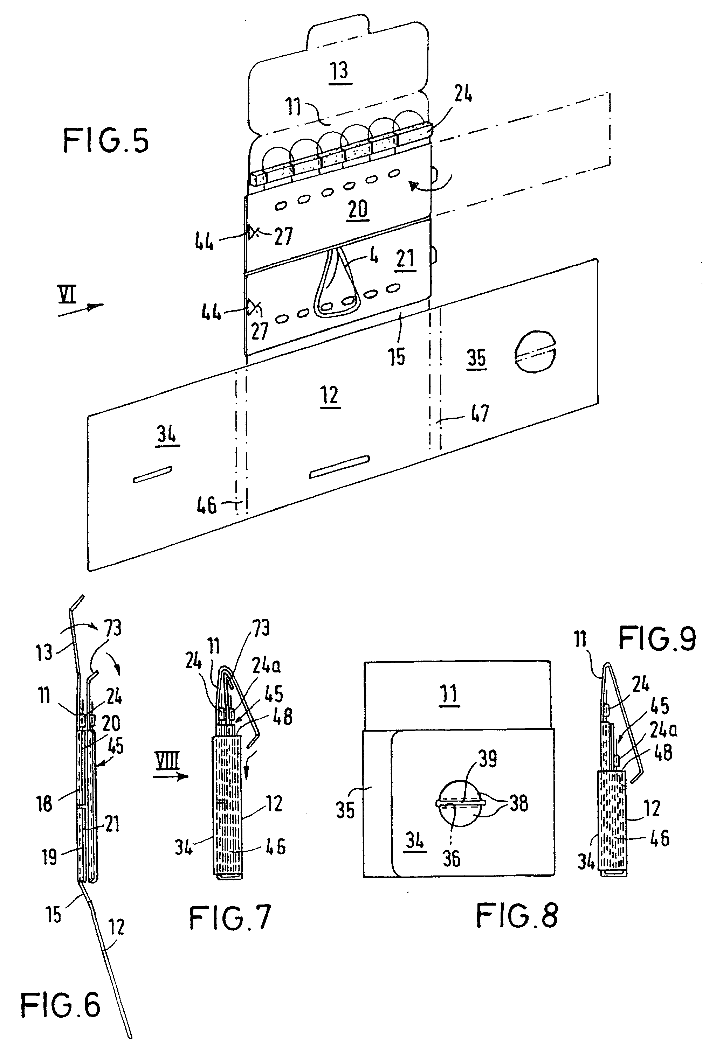

Nachdem sämtliche Fadenwickel verlegt und fixiert sind, werden die Wickeldorne aus den Löchern 40,41 herausgezogen und vor die erste Platte 11 mit den darübergeklappten Trennplatten können weitere Nahtmaterialträger 45, die später erläutert werden, vorgesetzt werden. Dann wird die zweite Platte 12 über den weiteren Nahtmaterialträger 45 gefaltet (Fig. 7). Man erkennt, daß die zweite Platte 12 die in der ersten Platte 11 vorgesehene Nadelhaltevorrichtung 24 freiläßt.

Dann wird zunächst die Verschlußplatte 35 hinter die erste Platte 11 gefaltet und die Verschlußplatte 34 wird hinter die Verschlußplatte 35 gefaltet, wobei die beiden Flügel 38 durch den Schlitz 36 hindurchgesteckt und anschließend auseinandergefaltet werden. Fig. 8 zeigt die Rückseite der Packung, wobei die Verschlußplatte 34 die Außenwand bildet. Sämtliche Platten 11,34,35, die in Fig. 1 von der Vorderseite dargestellt sind, sind in Fig. 8 von ihrer Rückseite sichtbar.

Die Verschlußplatten 34,35 sind mit der zweiten Platte 12 durch Stege 46,47 verbunden, welche ähnlich wie der Steg 15 durch Faltlinien oder Einschnitte begrenzt sind. Wenn die zweite Platte 12 über die erste Platte 11 gefaltet ist und die Verschlußplatten 34,35 aneinander auf der Rückseite der Packung verriegelt sind, wird zwischen den Platten 11,12 eine Tasche 48 gebildet, in die der zusätzliche Nahtmaterialträger 45 eingesteckt ist. In Fign. 6 und 7 kann die Nahtmateriallage 45 mit Hilfe der Lasche 73 herausgezogen werden.

Fig. 9 zeigt in gleicher Darstellung wie Fig. 7 ein geändertes Ausführungsbeispiel, bei dem die zweite Platte 12 kürzer ausgebildet ist als beim ersten Ausführungsbeispiel, so daß sie im umgefalteten Zustand nicht bis in die Nähe der Nadelhaltevorrichtung 24 reicht, sondern in größerem Abstand davon endet. Dieser Nahtmaterialträger 45 weist eine eigene Nadelhaltevorrichtung 24a auf und er endet unterhalb der Nadelhaltevorrichtung 24 der Packung. Daher liegen beide Nadelhaltevorrichtungen 24,24a frei, so daß der Nahtmaterialträger 45 nicht herausgezogen werden muß, um Zugriff zu den Nadeln des Nahtmaterialträgers 24 zu erhalten, d.h. beide Nadelreihen werden präsentiert.

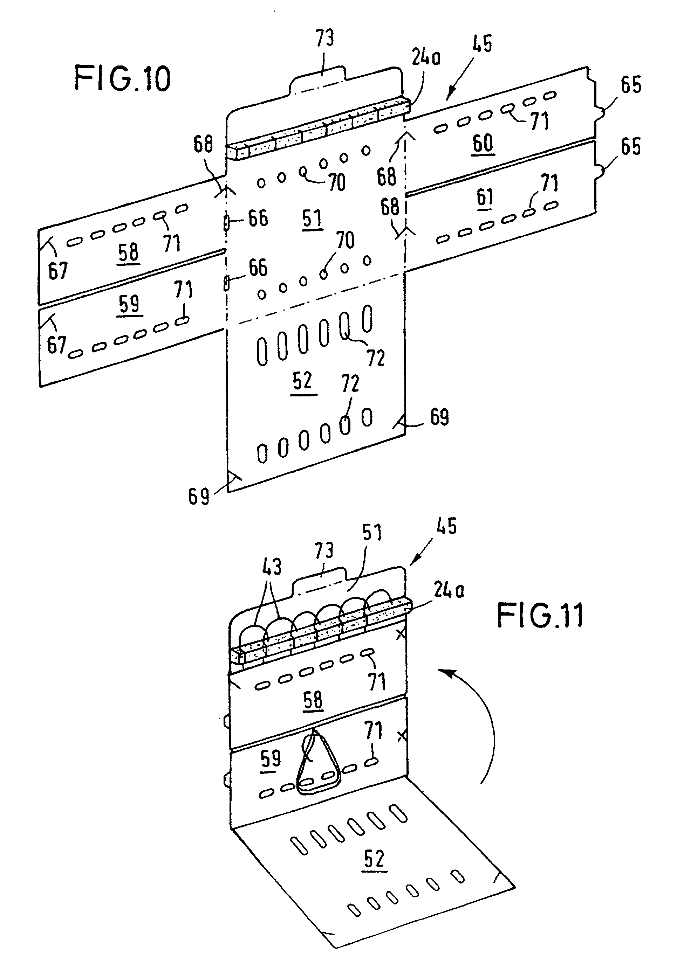

In den Fign. 9-11 ist der zusätzliche Nahtmaterialträger 45 dargestellt, der in die Tasche 48 der Packung eingesteckt ist. Er weist ebenfalls eine erste Platte 51 und eine über die erste Platte faltbare zweite Platte 52 auf. Seitlich neben der ersten Platte 51 sind auf jeder Seite zwei Trennplatten 58,59 bzw. 60,61 angeordnet. In der ersten Platte 51 sind zwei Reihen von Löchern 70 zum Hindurchstecken von Wickeldornen vorgesehen und jede der Trennplatten 58-61 enthält eine Reihe von entsprechenden Langlöchern 71, die nach dem Umfalten der Trennplatte mit den Löchern 70 einer Lochreihe der ersten Platte 51 zur Deckung kommen.

Über dem Bereich, der über den Trennplatten 58-61 angeordnet ist und der von der umgefalteten zweiten Platte 52 nicht bedeckt wird, befindet sich eine Nadelhaltevorrichtung 24a in Form eines querlaufenden Schaumstoffstreifens, an dem Nadeln befestigt werden können.

Ferner sind Verriegelungselemente in Form von Schlitzen 66 und Laschen 65 vorgesehen, um die Trennplatten 60,61 im zugeschlossenen Zustand zu verriegeln. Weitere Verriegelungsvorrichtungen in Form von Schrägschlitzen 67 und umgekehrt-V-förmigen Schlitzen 68 sind vorgesehen, um die Trennplatten 58,59 im geschlossenen Zustand zu verriegeln.

Schließlich sind an der zweiten Platte 52 Verriegelungselemente in Form von Schrägschlitzen 69 vorgesehen, die nach dem Schließen mit den Schlitzen 68 der ersten Platte 51 verriegelt werden, um die zweite Platte 52 im geschlossenen Zustand zu fixieren.

Die zweite Platte 52 ist mit zwei querlaufenden Reihen längsgerichteter Langlöcher 72 versehen, die über den Löchern 70,71 zu liegen kommen. Dadurch ist es möglich, die zweite Platte 52 zu schließen, während der Nahtmaterialträger 45 noch auf den Wickeldornen sitzt. An der ersten Platte 51 ist eine umfaltbare Lasche 73 vorgesehen, um den gleichformatigen Nahtmaterialträger 45 aus der Tasche der Packung herausziehen zu können.

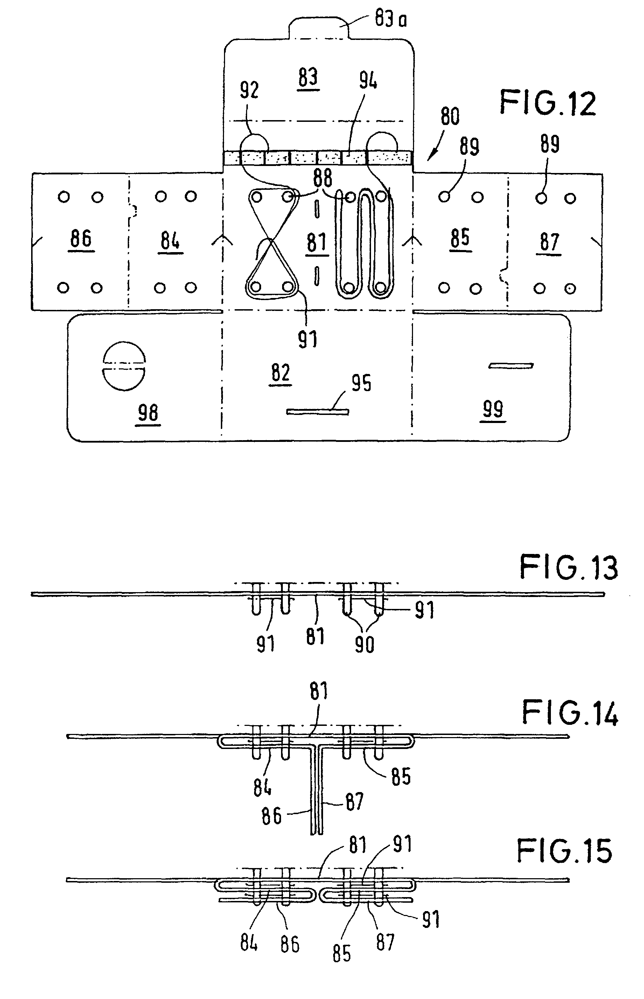

Der Nahtmaterialträger 45 unterscheidet sich von der in Fig. 1 dargestellten Packung im wesentlichen dadurch, daß er keine Verschlußplatten 34,35 aufweist. Das Herstellen der einzelnen Fadenwickel und das Überdecken mit den Trennplatten wird bei dem Nahtmaterialträger in gleicher Weise durchgeführt wie bei der Packung. Fig. 11 zeigt den Nahtmaterialträger im verschlußfertigen Zustand kurz vor dem Umfalten der zweiten Platte 52 auf die obenliegenden Trennplatten 58,59. Die Nadeln 43 sind an der Nadelhaltevorrichtung 24a fixiert und liegen zum Herausziehen frei. Wenn eine Nadel 43 herausgezogen wird, folgt der entsprechende Faden nach, ohne sich mit anderen Fäden zu verwickeln.

In den Fign. 12-18 ist eine weitere Packung 80 für chirurgisches Nahtmaterial dargestellt. Diese weist einen Zuschnitt aus Karton auf, mit einer ersten Platte 81, die durch eine Faltlinie mit einer zweiten Platte 82 verbunden ist und durch eine dazu parallele weitere Faltlinie mit einer Deckplatte 83. Die Platten 81,82,83 liegen in Längsrichtung hintereinander. An jeder Seite der ersten Platte 81 ist eine Trennplatte 84 bzw. 85 angeordnet, die im umgefalteten Zustand die halbe Breite der ersten Platte 81 bedecken kann. An jede Trennplatte 84 schließt sich eine Rückfaltplatte 86 bzw. 87 an.

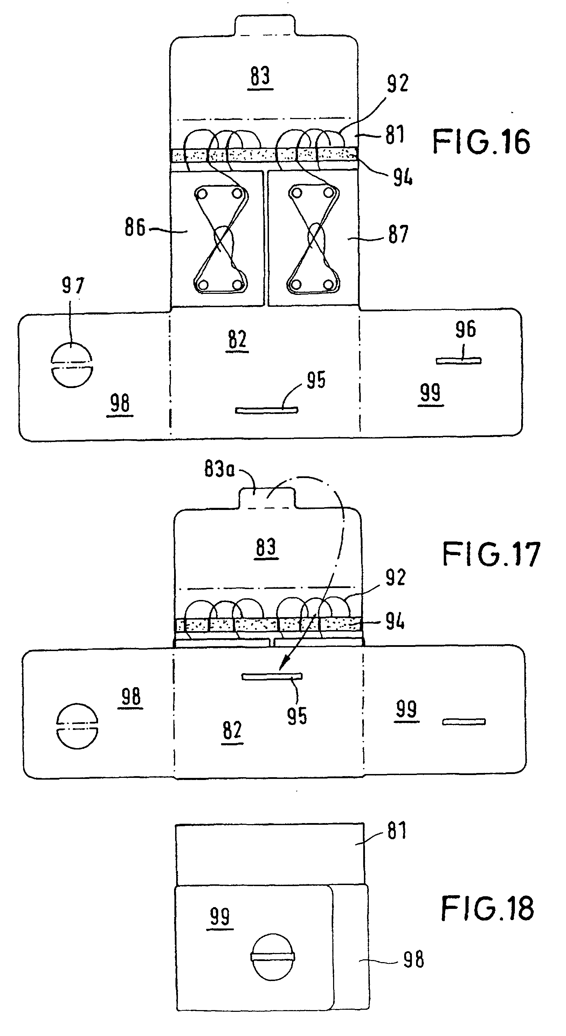

In der ersten Platte 81 sind Löcher 88 vorgesehen, durch die Wickeldorne 90 hindurchgesteckt werden können, um einen Fadenwickel 91 aus dem Nahtmaterial zu bilden. Eine oder zwei Nadel (n) 92 werden an der aus Schaumstoff bestehenden Nadelhaltevorrichtung 94 fixiert. Nachdem auf der linken Hälfte und der rechten Hälfte der ersten Platte 81 zwei Fadenwickel 91 gebildet worden sind, werden die Trennplatten 84 und 85 umgefaltet (Fig. 14) und auf den Rückseiten der Trennplatten 84,85 können weitere Fadenwickel 91 gewickelt werden (Fig. 15). Dann werden die Rückfaltplatten 86,87 über die jeweilige Trennplatte 84,85 gefaltet (Fig. 15), wodurch diese Fadenwickel vollständig bedeckt und fixiert werden. Dieser Zustand ist auch in Fig. 16 dargestellt, wobei auf die Rückfalteplatte 86,87 zusätzlich Nahtmaterialwickel positioniert werden können.

Es ist nicht erforderlich, die Rückfaltplatten 86,87, wie in Fig. 14 dargestellt, rechtwinklig abstehen zu lassen, während die Fadenwickel 91 auf den Trennplatten 84,85 gewickelt werden. Jede Rückfaltplatte 86,87 kann auch während des Wickelvorganges flach liegen und dann um 180° umgefaltet werden.

An die zweite Platte 82 schließen sich seitlich zwei Verschlußplatten 98,99 an, die mit zusammengreifenden Verschlußteilen 96,97 versehen sind. Wenn die zweite Platte 82 über die Rückfaltplatten 86,87 gefaltet ist (Fig. 17), werden die Verschlußplatten 98,99 um die Seitenkanten der ersten Platte herumgefaltet und mit ihren Verschlußteilen 96,97 auf der Rückseite der ersten Platte miteinander verriegelt. Fig. 18 zeigt die Rückansicht der geschlossenen Packung 90. Die Deckplatte 83 wird über die Vorderseite der ersten Platte 81 heruntergeklappt und mit ihrer Lasche 83a in einem Schlitz 95 der zweiten Platte 82 verriegelt. Die Deckplatte 83 bedeckt also die Nadelhaltevorrichtung 94 mit den daran befestigten Nadeln.

Zum Entnehmen von Nahtmaterial wird die Deckplatte 83 aufgeklappt. Die Nadeln 92 liegen dann zum Entnehmen frei und die einzelnen Fäden können durch Ziehen an der jeweiligen Nadel 92 herausgezogen werden.

Es ist auch möglich, bei der Packung 80 einen separaten Nahtmaterialträger in die von der Packung gebildete Tasche einzustecken und so mehrere Nahtmateriallagen übereinander oder hintereinander verschoben zu bilden.

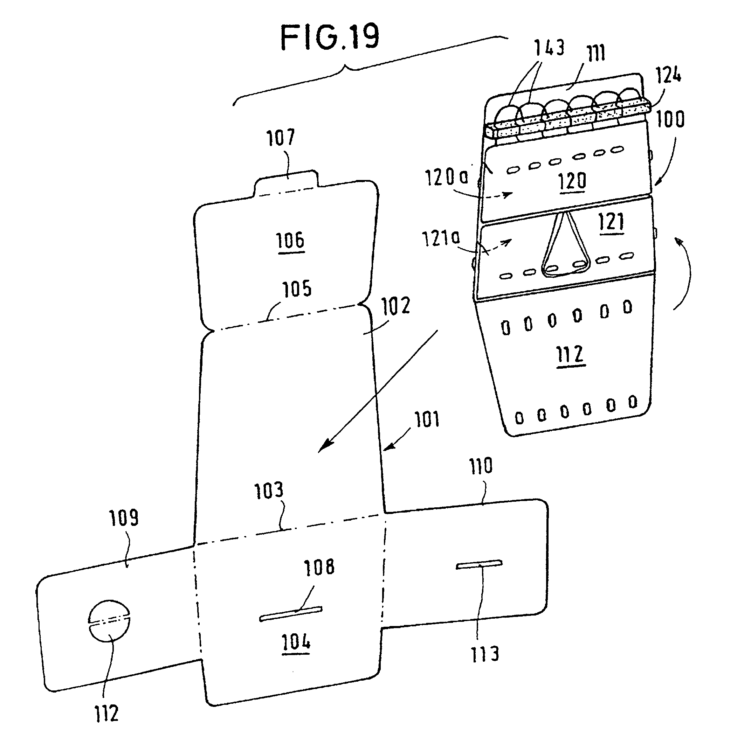

Bei dem Ausführungsbeispiel der Fign. 19-22 ist ein Nahtmaterialträger 100 vorgesehen, der in einer Tasche 101 untergebracht wird. Der Nahtmaterialträger 100 bildet eine Packung, die das Nahtmaterial enthält. Er weist eine erste Platte 111 auf, die bei dem vorliegenden Ausführungsbeispiel trapezförmig ausgebildet ist und sich zu demjenigen Endbereich, an dem die Nadelhaltevorrichtung 124 angeordnet ist, verjüngt. Die Breite der ersten Platte 111 nimmt also von unten nach oben hin ab. An den beiden schrägen Seitenkanten sind Trennplatten angelenkt, von denen in Fig. 19 nur die beiden oberen Trennplatten 120,121 sichtbar sind, die von rechts über die erste Platte 111 gefaltet wurden. Unter den Trennplatten 120,121 befinden sich die links angelenkten Trennplatten 120a und 121a, die von den Trennplatten 120,121 überdeckt werden. Jede der Trennplatten erstreckt sich nur über die halbe Höhe des Bedeckungsbereichs der Fadenwickel. Zusätzlich kann eine weitere Abdeckplatte 112 angebracht sein.

Die Tasche 101, die in Fig. 19 im auseinandergefalteten Zustand dargestellt ist, weist eine Grundplatte 102 auf, die im wesentlichen deckungsgleich mit der ersten Platte 111 des Nahtmaterialträgers 100 ist und die ebenfalls trapezförmig gestaltet ist. An die breite Seite der Trapezform schließt sich über eine Faltlinie 103 eine Deckplatte 104 an, die imstande ist, die Trennplatte 120,121 zu überdecken, wobei die Nadelhaltevorrichtung 124 jedoch freibleibt. An dem gegenüberliegenden Ende der Grundplatte 102 ist über eine Faltlinie 105 die Deckplatte 106 angelenkt, die mit einer Lasche 107 versehen ist, welche in einen Schlitz 108 der Deckplatte 104 eingesteckt werden kann. Von den beiden Seiten der Deckplatten 104 stehen Verschlußplatten 109,110 nach entgegengesetzten Richtungen ab. Diese haben zusammengreifende Verschlußelemente 112,113, mit denen sie auf der Rückseite der Grundplatte 102 gegenseitig verriegelt werden können.

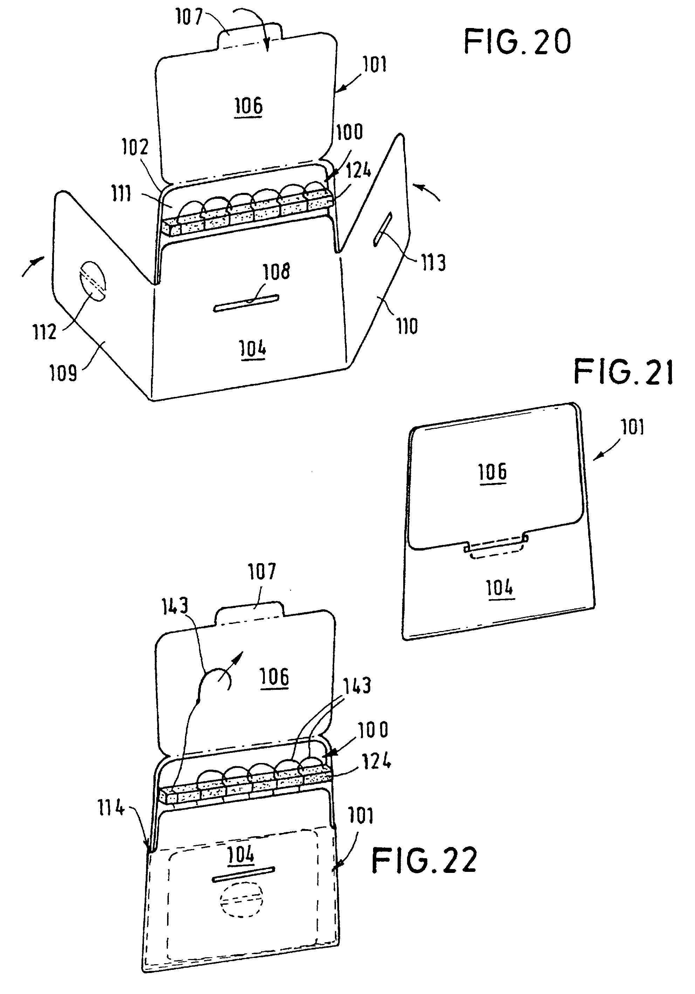

Fig. 22 zeigt den Nahtmaterialträger 100, der passend auf der Grundplatte 102 der Tasche 101 liegt, wobei die Deckplatte 104 über die Trennplatten gefaltet sind, so daß diese nicht sichtbar sind. Die Nadelhaltevorrichtung 124 wird jedoch von der Deckplatte 104 freigelassen. Die Verschlußplatten 109,110 werden hinter der Grundplatte 102 übereinandergefaltet und mit Hilfe der Verriegelungselemente 112,113 verriegelt. Dann wird die Deckplatte 106 über die Deckplatte 104 gefaltet, wobei die Lasche 107 in den Schlitz 108 eingesteckt wird.

Fig. 21 zeigt die Tasche 101 im geschlossenen Zustand und Fig. 22 zeigt die Tasche 101, nachdem die Deckplatte 106 hochgeklappt wurde, in dem Zustand zur Entnahme der Nadel 143 mit den daran befestigten Fäden. Man erkennt ferner, daß die Nadelhaltevorrichtung 124 freiliegt, und daß der Nahtmaterialträger 100 aus der Tasche 101 nicht herausgezogen werden kann, weil die Taschenöffnung 114 infolge der Trapezform der Tasche 101 eine geringere Breite hat als der darunterliegende Teil der Tasche und dessen Nahtmaterialträger 100. Der Nahtmaterialträger 100 kann also nicht aus der Tasche 101 herausgezogen werden.

Eine andere Möglichkeit der Befestigung des Nahtmaterialträgers in der Tasche würde darin bestehen, die Rückseite der ersten Platte 111 an der Grundplatte der Tasche, z.B. durch Klebepunkte, zu fixieren.

Die dargestellten Ausführungsbeispiele zeigen jeweils Nadelhaltevorrichtungen 24,24a,94, die aus einem horizontalen Schaumstoffstreifen bestehen. Es kann zweckmäßig sein, anstelle eines horizontalen Schaumstoffstreifens mehrere längliche vertikale Schaumstoffstreifen mit gegenseitigen Abständen vorzusehen, in die die Nadeln eingesteckt werden können.

## Patentansprüche

1. Mehrfäden-Packung für chirurgisches Nahtmaterial mit
einem einstückigen Kartonzuschnitt, der eine erste Platte (11;51;81;111) aufweist,
Trennplatten (18-21;58-61;84,85;120,121;120a, 121a), die über die erste Platte legbar sind und jeweils mindestens einen Faden des Nahtmaterials überlagern und mindestens einen anderen Faden unterlagern,
einer an der ersten Platte (11;51;81;111) angeordneten Nadelhaltevorrichtung (24;24a;94;124), die von den über die erste Platte gelegten Trennplatten nicht überdeckt wird,
**dadurch gekennzeichnet,**
**dass** die erste Platte (11;51;81;111) eine Wickelplatte für Nahtmaterial bildet und dass die Trennplatten (18-21;58-61;84,85;120,121;120a,121a) an gegenüberliegenden Kanten der ersten Platte faltbar angeordnet sind.

2. Mehrfäden-Packung nach Anspruch 1, **dadurch gekennzeichnet, daß** an jeder der beiden entgegengesetzten Kanten der ersten Platte (11;51;111) mindestens zwei Trennplatten (18-21;58-61;120,121; 120a,121a) angeordnet sind, wobei jeweils zwei einander gegenüberliegende Trennplatten auf der ersten Platte (12;52,112) übereinandergefaltet sind.

3. Mehrfäden-Packung nach Anspruch 1, **dadurch gekennzeichnet, daß** die Trennplatten (84,85) sich jeweils nur über einen Teil der ersten Platte (81) erstrecken und mit entsprechenden Rückfaltplatten (86,87) verbunden sind.

4. Mehrfäden-Packung nach einem der Ansprüche 1-3, **dadurch gekennzeichnet, daß** die Trennplatten (18-21;58-61;84-87;120,121;120a,121a) Löcher (41;71;89) mit geschlossenen Lochrändern aufweisen, die die Löcher der ersten Platte überlagern.

5. Mehrfäden-Packung nach Anspruch 4, **dadurch gekennzeichnet, daß** die Löcher (41;71,72) der Trennund/oder Abdeckplatten gleich groß oder größer sind als diejenigen (40,70,88) der ersten Platte.

6. Mehrfäden-Packung nach einem der Ansprüche 1-5, **dadurch gekennzeichnet, daß** eine mit der ersten Platte (11;51;81,111) verbundene zweite Platte (12;52;82,112) vorgesehen ist, die an dem der Nadelhaltevorrichtung (24;24a;94,124) abgewandten Rand angrenzt und in dem Zustand, daß sie über die erste Platte gefaltet ist, die Nadelhaltevorrichtung (24;24a;94,124) freiläßt.

7. Mehrfäden-Packung nach Anspruch 6, **dadurch gekennzeichnet, daß** die zweite Platte (12;82) an entgegengesetzten Kanten mit Verschlußplatten (34,35; 98,99) verbunden ist, die im umgefalteten Zustand die Außenseite der ersten Platte überlagern und zusammengreifende Verriegelungselemente (36,37;98,99) aufweisen, wobei die erste und die zweite Platte eine Tasche (48) umschließen.

8. Mehrfäden-Packung nach Anspruch 7, **dadurch gekennzeichnet, daß** die Tasche (48) einen herausziehbaren separaten Nahtmaterialträger (45) mit Seitenund Abdeckklappen enthält, der seinerseits eine Mehrfäden-Packung nach einem der vorhergehenden Ansprüche bildet.

9. Mehrfäden-Packung nach Anspruch 8, **dadurch gekennzeichnet, daß** der Nahtmaterialträger (45) eine eigene Nadelhaltevorrichtung (24a) aufweist und die Nadelhaltevorrichtung (24) der ersten Platte (11,81) nicht bedeckt, und daß die zweite Platte (12,82) auch die Nadelhaltevorrichtung (24a) des in die Tasche (48) eingesteckten separaten Nahtmaterialträgers (45) freiläßt.

10. Mehrfäden-Packung nach einem der Ansprüche 1-5, mit einem Nahtmaterialträger (100), der in einer separaten Tasche (101) enthalten und fixiert ist.

11. Mehrfäden-Packung nach Anspruch 10, **dadurch gekennzeichnet, daß** die Breite der Tasche (101) und des darin enthaltenen Nahtmaterialträgers (100) sich zur Taschenöffnung (114) hin verringert, wodurch das Herausziehen des Nahtmaterialträgers (100) aus der Taschenöffnung (114) verhindert wird.

## Claims

1. Multithread package for surgical sewing material comprising
a single-piece carton blank comprising a first panel (11; 51; 81; 111),
separation panels (18-21;58-61;84,85;120,121;120a,121a) arranged for folding over said first panel and each lying over at least one thread of said sewing material and lying under at least one further thread,
a needle supporting means (24;24a;94;124)arranged at said first panel (11;51;81;111), said means not being covered by said separation panels folded over said first panel,
**characterized in that**
the first panel (11;51;81;111) forms a winding plate for suture material and the separation panels (18-21;58-61;84,85;120,121;120a,121a) are arranged for folding at opposite edges of the first panel.

2. Multithread package according to claim 1, wherein at least two separation panels (18-21;58-61; 120,121; 120a,121a) are arranged at each of said two opposite edges of said first panel (11;51;111), two separation panels opposite each other being folded over each other on said first panel (12;52,112).

3. Multithread package according to claim 1, wherein said separation panels (84,85) each only extend across a part of said first panel (81) and are connected to corresponding backfolding panels (86,87).

4. Multithread package according to one of claims 1-3, wherein said separation panels (18-21;58-61;120,121;120a,121a) comprise holes (47;71;89) with closed hole rims superimposed over said holes of said first panel.

5. Multithread package according to claim 4, wherein said holes (47;71;89) of said separation and/or cover panels are as large as or larger than those (40,70,88) of said first panel.

6. Multithread package according to one of claims 1-5, wherein a second panel (12;52;82,112) connected to said first panel (11;51;81,111) is provided adjacent said edge opposite said needle supporting means (24;24a;94,124) and leaving exposed said needle supporting means (24;24a;94,124) in said state in which said second panel is folded over said first panel.

7. Multithread package according to claim 6, wherein said second panel (12;82) is connected, at opposite edges thereof, to closing panels (34,35;98,99) which, in a state of being folded over, are superimposed over the outer side of said first panel and comprise cooperative locking elements (36,37;98,99), the first and the second panel enclosing an envelope (48).

8. Multithread package according to claim 7, wherein the envelope (48) comprises an extractable separate sewing material carrier (45) with lateral and covering panels, which carrier itself provides a multithread package according to claim 1.

9. Multithread package according to claim 8, wherein the sewing material carrier (45) comprises a needle supporting means (24a) of its own and does not cover the needle supporting means (24) of the first panel (11,81), and in that the second panel (12,82) also leaves exposed the needle supporting means (24a) of the separate sewing material carrier (45) inserted into the envelope (48).

10. Multithread package according to one of claims 1-5, having a sewing material carrier (100) contained and secured in a separate envelope (101).

11. Multithread package according to claim 10, wherein the width of the envelope (101) and of the sewing material carrier (100) contained therein decreases towards the envelope opening (114), whereby the extraction of the sewing material carrier (100) out of the envelope opening (114) is avoided.

## Revendications

1. Emballage de fils multiples pour matériel de suture chirurgical, avec
un flan découpé en carton, d'un seul tenant, qui comprend une première plaque (11 ; 51 ; 81 ; 111),
des plaques de séparation (18-21 ; 58-61 ; 84, 85 ; 120, 121 ; 120a, 121a) que l'on peut placer par-dessus la première plaque, et qui surmontent, chacune, par le dessus, au moins un fil du matériel de suture, et sont sous-jacentes, chacune, d'au moins un autre fil,
un dispositif de support d'aiguille (24 ; 24a ; 94 ; 124) qui est disposé sur la première plaque (11 ; 51 ; 81 ; 111) et qui n'est pas recouvert par les plaques de séparation placées par-dessus la première plaque,
**caractérisé**
**en ce que** la première plaque (11; 51; 81; 111) forme une plaque d'enroulement pour du matériel de suture, et en ce que les plaques de séparation (18-21 ; 58-61 ; 84, 85 ; 120, 121 ; 120a, 121a) sont agencées, de manière repliable, sur des bords opposés de la première plaque.

2. Emballage de fils multiples selon la revendication 1, **caractérisé en ce que** sur chacun des deux bords opposés de la première plaque (11 ; 51 ; 111) sont agencées au moins deux plaques de séparation (18-21 ; 58-61 ; 120, 121 ; 120a, 121a), deux plaques de séparation respectivement opposées l'une à l'autre étant à chaque fois repliées l'une sur l'autre sur la première plaque (12 ; 52, 112).

3. Emballage de fils multiples selon la revendication 1, **caractérisé en ce que** les plaques de séparation (84, 85) ne s'étendent chacune que sur une partie de la première plaque (81) et sont reliées à des plaques repliables en sens inverse, correspondantes (86, 87).

4. Emballage de fils multiples selon l'une des revendications 1-3, **caractérisé en ce que** les plaques de séparation (18-21 ; 58-61 ; 84-87 ; 120, 121 ; 120a, 121a) présentent des trous (41 ; 71 ; 89) avec des bords de trous fermés, qui surmontent par le dessus les trous de la première plaque.

5. Emballage de fils multiples selon la revendication 4, **caractérisé en ce que** les trous (41 ; 71, 72) des plaques de séparation et/ou de recouvrement sont de même grandeur ou sont plus grands que ceux (40, 70, 88) de la première plaque.

6. Emballage de fils multiples selon l'une des revendications 1-5, **caractérisé en ce qu'**il est prévu une seconde plaque (12 ; 52 ; 82, 112) qui est reliée à la première plaque (11 ; 51 ; 81, 111) et est adjacente au bord opposé à celui où se trouve le dispositif de support d'aiguille (24 ; 24a ; 94, 124), et qui dans l'état dans lequel elle est repliée par-dessus la première plaque, laisse dégagé le dispositif de support d'aiguille (24 ; 24a ; 94, 124).

7. Emballage de fils multiples selon la revendication 6, **caractérisé en ce que** la seconde plaque (12 ; 82) est reliée, sur des bords opposés, à des plaques de fermeture (34, 35 ; 98, 99) qui, dans l'état replié, surmontent le côté extérieur de la première plaque et présentent des éléments de verrouillage (36, 37 ; 96, 97) venant en prise réciproque, la première et la seconde plaque définissant une pochette (48).

8. Emballage de fils multiples selon la revendication 7, **caractérisé en ce que** la pochette (48) contient un support de matériel de suture séparé (45) qui peut être extrait et comporte des volets latéraux et de recouvrement, et qui forme pour sa part un emballage de fils multiples selon l'une des revendications précédentes.

9. Emballage de fils multiples selon la revendication 8, **caractérisé en ce que** le support de matériel de suture (45) comporte un support d'aiguille (24a), qui lui est propre, et ne recouvre pas le dispositif de support d'aiguille (24) de la première plaque (11, 81), et **en ce que** la seconde plaque (12, 82) laisse également dégagé le dispositif de support d'aiguille (24a) du support de matériel de suture séparé (45) inséré dans la pochette (48).

10. Emballage de fils multiples selon l'une des revendications 1-5, comprenant un support de matériel de suture (100) contenu et fixé dans une pochette séparée (101).

11. Emballage de fils multiples selon la revendication 10, **caractérisé en ce que** la largeur de la pochette (101) et du support de matériel de suture (100) qu'elle contient, se réduit en direction de l'ouverture de pochette (114), en empêchant ainsi d'extraire le support de matériel de suture (100) de l'ouverture de pochette (114).
